# EUROPEAN PATENT APPLICATION

(11) **EP 4 697 352 A1**
(43) Date of publication of application: **18.02.2026**
(21) Application number: 25190466.0
(22) Date of filing: 18.07.2025
(51) Int. Cl.: G16H 40/63

(54) **SCREEN DISPLAY APPARATUS AND METHOD FOR MEDICAL DEVICE**

(30) Priority: 14.08.2024 KR 20240108707
(71) Applicant: VIOL Co. Ltd., Bundang-gu Seongnam-si, Gyeonggi-do, 13510 (KR)
(72) Inventor: JEON, Yeonggi, 13510 Seongnam-si, Gyeonggi-do (KR); LEE, Sangjin, 13510 Seongnam-si, Gyeonggi-do (KR)
(74) Representative: Zimmermann, Tankred Klaus

(57) **Abstract**

Disclosed are a screen display apparatus and method for a medical device. The disclosed apparatus includes a processor that controls an operation of a medical device based on a treatment parameter. The processor generates a first screen which presents a state object related to an operation of the medical device, presents the first screen on a display, generates a second screen which presents an adjustment object for adjusting a treatment parameter of the medical device when determining that an interaction event has been generated, presents the second screen on the display, adjusts a treatment parameter corresponding to a corresponding adjustment object based on an input value corresponding to a sensed interaction when sensing the interaction of a user with the adjustment object of the second screen, and controls an operation of the medical device based on the adjusted treatment parameter.

## Description

### [Technical Field]

The present disclosure relates to a screen display apparatus and method for a medical device, and more particularly, to a screen display apparatus and method for a medical device, which can display an object displayed on the display of a medical device (e.g., a skin treatment device) by adjusting the object.

### [Background Art]

As interest in beauty care is gradually increased, the number of consumers who try to undergo a skin treatment, for example, the treatment, care, or management of the skin is increasing. Accordingly, a skin treatment device that performs the treatment, care, or management of the skin is actively developed.

A conventional skin treatment device is based on a laser, a radio frequency, or ultrasonic waves. Furthermore, the conventional skin treatment device includes several modes in which skin characteristics are divided into several types through clinical trials in a development stage and the setting value of the skin treatment device is made different depending on each characteristic.

The setting value may include the output level, wavelength, frequency, and length of a focus of a laser, a radio frequency, or ultrasonic waves.

However, in a graphic user interface (GUI) that is displayed on the display of the conventional skin treatment device, basic information related to a treatment (called treatment information) and a manipulation button capable of changing a setting value coexist.

The use of the manipulation button is limited due to a limited space because the space of the GUI is limited. For example, it is necessary to look at the manipulation button for a long time or the manipulation button is incorrectly pressed because the size of the manipulation button is inevitably small due to the limited space and thus the manipulation button is rarely seen.

The contents described in the Background Art are to help the understanding of the background of the disclosure, and may include contents that are not a disclosed conventional technology.

### [Documents of Related Art]

### [Patent Document]

Korean Patent Application Publication No. 10-2016-0110894 (USER SPECIFIC SKIN CARE SYSTEM AND SKIN CARE METHOD USING THEREOF)
Korean Patent Application Publication No. 10-2022-0167261 (TREATMENT VIDEO DISPLAY DEVICE)

### [Summary of Invention]

### [Technical Problem]

An object of the present disclosure is to provide a screen display apparatus and method for a medical device, which highlight a visual effect of an object, such as a manipulation button that is displayed on the display of a medical device, such as a skin treatment device.

### [Solution to Problem]

A screen display apparatus for a medical device according to an embodiment of the present disclosure is a medical device including a handpiece for a treatment. The screen display apparatus includes a display, memory in which one or more treatment parameters related to the treatment are stored, and a processor configured to control an operation of the medical device based on the treatment parameter. The processor generates a first screen which presents a state object related to the operation of the medical device, controls the display so that the display presents the first screen, determines whether an interaction event for the medical device is generated, generates a second screen which presents an adjustment object for adjusting the treatment parameter of the medical device when determining that the interaction event has been generated, controls the display so that the display presentss the second screen, senses an interaction of a user with the adjustment object of the second screen, adjusts the treatment parameter corresponding to the adjustment object based on an input value corresponding to the sensed interaction, and controls the operation of the medical device based on the adjusted treatment parameter.

The processor may sense the proximity of the user and determine that the interaction event has been generated when sensing that the user has approached.

When determining that the interaction event has been generated, the processor may control the first screen to disappear from the display and control the second screen to be displayed on the display. In this case, the processor may adjust the size of the adjustment object of the second screen to be greater than the size of the state object of the first screen.

When determining that the interaction event has been generated, the processor may control the first screen and the second screen to be displayed on the display so that the first screen and the second screen are spaced apart from each other. In this case, the processor may adjust the size of the adjustment object of the second screen to be greater than the size of the state object of the first screen on the display and adjust the size of the second screen to be greater than the size of the first screen.

When determining that the interaction event has been generated, the processor may control the first screen and the second screen to be displayed on the display and control at least some area of the first screen to be overlaid with the second screen. In this case, the processor may adjust the size of the adjustment object of the second screen to be greater than the size of the state object of the first screen and adjust the size of the second screen to be greater than the size of the first screen.

The processor may display an edge of the second screen in a set color in response to the interaction event.

The processor may display the remaining area of the second screen except the edge of the second screen in a set color in response to the interaction event.

A plurality of input value selection buttons in which different input values have been set may be assigned to the adjustment object. The processor may provide the display with screen data of the plurality of input value selection buttons so that the plurality of input value selection buttons assigned to the adjustment object is displayed on the display when the adjustment object is selected.

When any one of the plurality of input value selection buttons is selected, the processor may adjust the treatment parameter corresponding to the adjustment object based on an input value set in the selected button.

A screen display method for a medical device according to an embodiment of the present disclosure is a screen display method performed by a screen display apparatus for a medical device including a handpiece for a treatment. The screen display method includes storing one or more treatment parameters related to the treatment, generating a first screen which presents a state object related to an operation of the medical device, controlling a display so that the display presents the first screen, determining whether an interaction event for the medical device is generated, generating a second screen on which an adjustment object for adjusting a treatment parameter of the medical device when determining that the interaction event has been generated, controlling the display so that the display presents the second screen, sensing an interaction of a user with the adjustment object of the second screen, adjusting the treatment parameter corresponding to the adjustment object based on an input value corresponding to the detected interaction, and controlling the operation of the medical device based on the adjusted treatment parameter.

### [Advantageous Effects of Invention]

As described above, according to embodiments of the present disclosure, when a user approaches the controller during a skin treatment through a medical device (e.g., a skin treatment device), the second screen that displays an adjustment object for adjusting a treatment parameter of the medical device is displayed on the display, but the adjustment object is visually highlighted and displayed. Accordingly, the adjustment object (e.g., a manipulation button) can be much easily manipulated. That is, the identification of the adjustment object can be further facilitated and a malfunction occurrence rate can be reduced by increasing the size of the adjustment object within the second screen.

Furthermore, when a user approaches the controller during a skin treatment through a medical device (e.g., a skin treatment device), the edge of the second screen displayed on the display is indicated in a set color or the remaining area of the second screen except the edge is indicated in a set color. Accordingly, the second screen and an adjustment object of the second screen can be visually highlighted. Accordingly, the user can quickly check where a manipulation button (i.e., the adjustment object) is currently located, and can easily manipulate the manipulation button (i.e., the adjustment object) without an erroneous manipulation because the size of the manipulation button is large.

Furthermore, when any one of adjustment objects of the second screen is selected (or touched), buttons by which an input value may be selected can be intuitively identified because a plurality of input value selection buttons assigned to the selected adjustment object is displayed on the display. Accordingly, there is an effect in that a desired input value can be input very easily and rapidly because the desired input value can be immediately selected and input.

### [Brief Description of Drawings]

FIG. 1 is a diagram for describing an example in which a medical device according to an embodiment of the present disclosure is used.
FIG. 2 is a block diagram for describing internal components of a controller illustrated in FIG. 1.
FIGS. 3 to 5 are diagrams exemplarily illustrating a second screen and an adjustment object according to an embodiment of the present disclosure.
FIG. 6 is a diagram for describing a method of manipulating an adjustment object according to an embodiment of the present disclosure.
FIGS. 7 and 8 are flowcharts for describing a screen display method for a medical device according to an embodiment of the present disclosure.

### [Description of Embodiments]

The present disclosure may be modified in various ways and may have various embodiments. Specific embodiments are to be illustrated in the drawings and to be described in the detailed description.

It is however to be understood that the present disclosure is not intended to be limited to the specific embodiments, but that the specific embodiments include all of modifications, equivalents and/or substitutions included in the spirit and technical scope of the present disclosure.

The terms used in this application are used to only describe specific embodiments and are not intended to restrict the present disclosure. An expression of the singular number should be construed as including an expression of the plural number unless clearly defined otherwise in the context. It is to be understood that in this application, a term, such as "include (or comprise)" or "have", is intended to designate the presence of a characteristic, a number, a step, an operation, a component, a part or a combination of them described in the specification and does not exclude the possible existence or addition of one or more other characteristics, numbers, steps, operations, components, parts or combinations of them in advance.

All terms used herein, including technical terms or scientific terms, have the same meanings as those commonly understood by a person having ordinary knowledge in the art to which the present disclosure pertains, unless defined otherwise in the specification. Terms, such as those defined in commonly used dictionaries, should be construed as having the same meanings as those in the context of a related technology, and are not construed as ideal or excessively formal meanings unless explicitly defined otherwise in the application.

Hereinafter, preferred embodiments of the present disclosure are more specifically described with reference to the accompanying drawings. In describing the present disclosure, in order to help general understanding, the same reference numerals are used to denote the same components throughout the drawings, and a redundant description of the same components is omitted.

In the following description, it is assumed that a medical device is a skin treatment device.

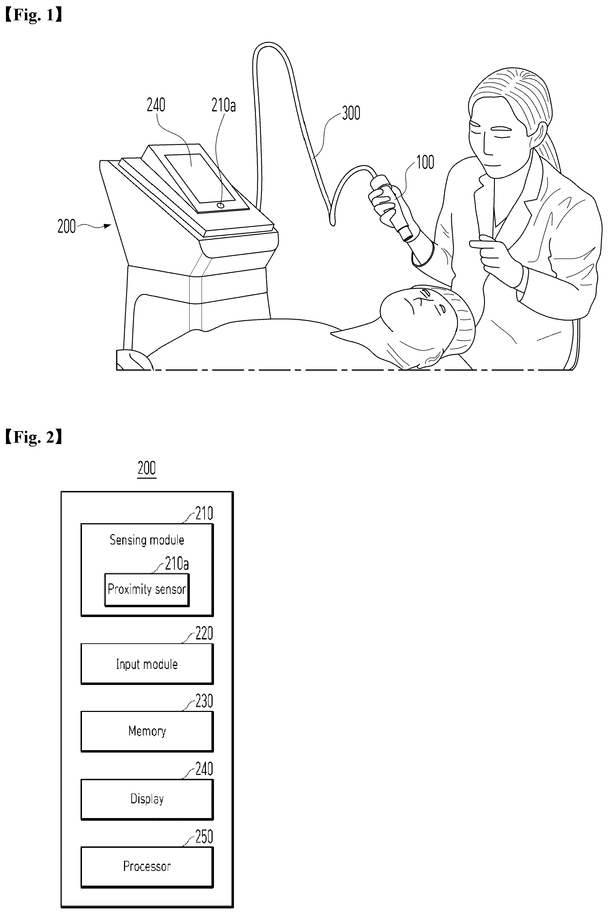

FIG. 1 is a diagram for describing an example in which a medical device according to an embodiment of the present disclosure is used.

The medical device according to an embodiment of the present disclosure may include a handpiece 100 and a controller 200.

In general, the handpiece 100 has a deformed cylindrical shape which can be easily held with a hand, but the present disclosure is not essentially limited thereto. The handpiece may have various shapes.

The handpiece 100 may include a skin treatment part. The skin treatment part is connected to the end of the handpiece 100, and may treat the skin. The skin treatment part may include a needle part (or a tip part) having a sharp needle shape, but the present disclosure is not essentially limited thereto. The skin treatment part may have various structures if the skin treatment part can treat the skin. For example, the skin treatment part may have a structure capable of a high-intensity focused ultrasound (HIFU), galvanic, non-invasive, and invasive radio frequency (RF) treatment.

The controller 200 is connected to the handpiece 100 through a cable 300 having a predetermined length. That is, one end of the cable 300 may be connected to the controller 200. The other end of the cable 300 may be connected to the handpiece 100. For example, when the handpiece 100 is held in a holder (not illustrated), the cable 300 will be down compared to a case in which the handpiece is used in a skin treatment.

The controller 200 displays information on handpiece 100 or information on treatments by the handpiece 100 on (screen, i.e., display 240) of the controller 200. The controller may control operations related to the screen of the controller 200. For this reason, the controller 200 may also be referred to as a screen display device.

A display 240 may be installed at the top of the controller 200.

The display 240 may display (or present) various objects related to a skin treatment, for example.

If necessary, the display 240 may include a touch screen (e.g., an LCD touch screen) having a predetermined size. Accordingly, the display 240 may transmit a touch input of a user to a processor 250.

Accordingly, it may be seen that the display 240 also has a function for transmitting a touch input of a user in addition to a function for displaying an object. In an embodiment of the present disclosure, the display 240 may be called a manipulation panel.

One or more proximity sensors 210a may be installed at the top (more specifically, around the display 240) of the controller 200. The proximity sensor 210a may sense whether at least body part of a user approaches.

FIG. 2 is a block diagram for describing internal components of the controller 200 illustrated in FIG. 1.

The controller 200 may include a sensing module 210, an input module 220, memory 230, the display 240, and the processor 250.

The sensing module 210 may be configured to sense an interaction event by a user. In this case, the interaction event is an event in which the user shows his or her intention of manipulating the controller 200 through the display 240, and may be detected directly and indirectly through the proximity of the user or the holding of the handpiece 100.

For example, the sensing module 210 includes the proximity sensor 210a, and may sense whether at least body part (e.g., a hand or a finger) of a user (e.g., a doctor) approaches through the proximity sensor 210a.

The type of proximity sensor 210a may be various. In an embodiment of the present disclosure, any type of proximity sensor may be used because the proximity sensor has only to sense the proximity of a user.

The input module 220 may detect an interaction (e.g., a touch signal) of a user with an adjustment object displayed on the display 240.

For example, the display 240 may display adjustment objects for adjusting the treatment parameter of the medical device. A user may input the value of a desired treatment parameter by interacting with a desired adjustment object on the display 240. The input module 220 may detect the value of the desired treatment parameter by detecting the interaction of the user. In this case, input value selection buttons corresponding to predetermined candidate input values may be displayed in the adjustment object or a visual object (e.g., a sliding bar or an arrow) for adjusting or changing input values may be displayed in the adjustment object, but embodiments of the present disclosure are not limited thereto.

In this case, when the desired adjustment object is selected (or touched), a touch signal corresponding to the desired adjustment object is generated by the input module 220 and applied to the processor 250. Accordingly, the touch signal according to the selection (or touch) of the desired adjustment object may be an example of the interaction of the user with the adjustment object.

Furthermore, when any one of a plurality of input value selection buttons is selected, the input module 220 may generate a signal (e.g., may be called a button identification signal) that provides notification that the selected button corresponds to any button and apply the signal to the processor 250. In this case, the button identification signal may be another example of the interaction of the user with the adjustment object.

The memory 230 may store data necessary for an operation of the controller 200.

The memory 230 may store one or more treatment parameters related to a treatment.

Furthermore, the memory 230 may store a program including instructions for performing a series of operations that are performed by the controller 200.

The memory 230 may include a nonvolatile memory device or a volatile memory device. According to an embodiment, the memory 230 may be included in the processor 250, but the present disclosure is not limited thereto.

The display 240 may display a screen on which an object related to an operation of the medical device is displayed. The object is generated by the processor 250. Furthermore, image or video data for indicating the object may be transmitted from the processor 250 to the display 240.

In this case, the object may include an adjustment object and a state object.

The adjustment object is an object for adjusting a treatment parameter related to an operation of the medical device. A user may adjust a treatment parameter corresponding to the adjustment object by interacting with the adjustment object. For example, the adjustment object may include a treatment adjustment object for adjusting a parameter related to the intensity of a treatment. A user may change the intensity of the treatment by touching the treatment adjustment object.

For example, the adjustment object may be generated to respond to the touch of a user and to have its value, such as a shot intensity change, a depth change, a spot size change, or a cooling condition change, changed.

In contrast, the state object may be an object indicative of information related an operating state of the medical device. For example, the state object is an object not related to a treatment parameter. Although a user interacts with the state object, the treatment parameter may not be changed.

For example, the state object may indicate information that does not need to be manipulated by a user, such as patient information, treatment information, tip information, and current setting information (e.g., shot intensity or a depth).

In this case, a screen corresponding to an area including the state object may be denoted as a first screen. A screen corresponding to an area including the adjustment object may be denoted as a second screen.

In FIG. 2, the input module 220 and the display 240 have been illustrated as separated components, but the input module 220 and the display 240 may be considered to have been integrated into one module.

The processor 250 may control an operation of the medical device based on a treatment parameter. According to an embodiment, the processor 250 may control an operation of the medical device by executing a program (or an application) including at least one instruction stored in the memory 230 and performing operations corresponding to the program based on the results of the execution.

For example, the processor 250 may include integrated circuit elements, such as a central processing unit (CPU), a graphical processing unit (GPU), an application specific integrated circuit (ASIC), a field programmable gate array (FPGA), and a micro controller unit (MCU), but the present disclosure is not limited thereto.

The processor 250 may control the display 240 to display a screen that displays (or includes) an object. For example, the processor 250 may generate screen data corresponding to a screen on which objects are disposed, and may enable the screen to be output through the display 240 by outputting the screen data to the display 240.

For example, the processor 250 may control the display 240 so that the display 240 displays (presents) the first screen and/or the second screen.

According to embodiments of the present disclosure, when a user does not manipulate the medical device (i.e., when an interaction event is not generated), the display 240 may display the first screen on which the state object has been displayed. When a user wants to manipulate the medical device (i.e., when an interaction event is generated), the display 240 may display the second screen on which the adjustment object has been displayed.

In this case, the second screen may be visually more highlighted than the first screen on the display 240. In this case, "visually highlighting" includes that a more visual effect is assigned to the second screen and also includes that a less visual effect is assigned to the first screen. For example, "visually highlighting" also includes that the first screen disappears.

Information related to the medical device is quite a lot. When a large amount of information is displayed on one display, the size of an object corresponding to each of pieces of information is reduced. In this case, it may be difficult for a user to interact with objects for manipulating treatment parameters of the medical device. In contrast, according to embodiments of the present disclosure, a user can easily manipulate a treatment parameter of the medical device because the adjustment object is displayed when the user tries to manipulate the medical device.

The processor 250 may determine whether an interaction event of a user has been generated, and may control the display 240 to generate the second screen on which adjustment objects are displayed based on the results of the determination. The generation of the interaction event of the user means that the user tries to manipulate or control the medical device. Accordingly, the processor 250 may control the display 240 to display the second screen on which the adjustment objects are displayed so that the user can control and manipulate the medical device more easily.

For example, the processor 250 may determine whether a user has approached the controller 200, and may determine that an interaction event has been generated if the user has approached the controller 200. To this end, the processor 250 may determine whether the interaction event has been generated based on a sensing signal from the sensing module 210. That is, the processor 250 may sense the proximity of the user through the proximity sensor 210a of the sensing module 210, and may determine that the interaction event of the user has been generated when sensing that the user (e.g., a doctor) has approached the controller 200. In this case, "approach" may mean that at least body part (e.g., a hand or a finger) of the user approaches within a set distance. Accordingly, the generation of the interaction event may mean that the user has approached the controller 200, but the user's behavior to touch the display 240 has not been performed. The proximity sensor 210a has been described as being included in the sensing module 210, but the sensing module 210 or the proximity sensor 210a may be considered to be included in the processor 250, if necessary.

In the above description, whether an interaction event has been generated is determined by using the proximity sensor 210a. However, whether an interaction event has been generated may be determined by using another component other than the proximity sensor 210a.

Near field communication (NFC) may be used as an example of another component other than the proximity sensor 210a. For example, a construction in which an NFC tag (not illustrated) has been installed in the handpiece 100 and an NFC reader (not illustrated) has been installed in a holder (not illustrated) may be assumed. In such an NFC construction, when the handpiece 100 is held in the holder, the NFC reader of the holder may read information (i.e., tagging information) of the NFC tag of the handpiece 100. The information of the NFC tag read as described above is applied to the processor 250. The processor 250 may determine that the handpiece 100 has been held in the holder. Accordingly, the processor 250 may determine that an interaction event of a user (e.g., a doctor) has been generated because the processor 250 may consider that the user has approached the controller 200 in order to manipulate an object on the display 240 during a treatment.

A gyro sensor may be used as another example of another component other than the proximity sensor 210a. For example, when a gyro sensor (not illustrated) is installed in the handpiece 100, the processor 250 may calculate the degree of inclination of the handpiece 100 based on a sensing value (e.g., an angular speed) from the gyro sensor. The processor 250 may sense that a user (e.g., a doctor) approaches the controller 200 based on the calculated degree of inclination of the handpiece 100. If a skin treatment is being performed on a patient, the degree of inclination of the handpiece 100 will be great because the handpiece 100 needs to be directed toward the face of the patient. If a user temporarily stops a skin treatment and approaches the controller 200 in order to manipulate an object on the display 240, the degree of inclination of the handpiece 100 will be small because the handpiece 100 is made horizontal or the skin treatment part connected to the end of the handpiece 100 is made upward. When the degree of inclination of the handpiece 100 is small as described above, the processor 250 may determine that an interaction event of the user has been generated.

A tension sensor that measures the tension of the cable 300 may be used as still another example of another component other than the proximity sensor 210a. For example, when a user (e.g., a doctor) approaches the controller 200 with the handpiece 100, the cable 300 connected to the handpiece 100 will more droop compared to a case in which a skin treatment is performed. The tension of the cable 300 will also be different because the degree that the cable 300 droops when the skin treatment is performed as described above and the degree that the cable 300 droops when the user temporarily stops the skin treatment and approaches the controller 200 are different. Accordingly, the processor 250 may measure the tension of the cable 300 connected to the handpiece 100, and may determine that an interaction event of the user has been generated when the measured tension is within a set range.

When determining that the interaction event has been generated, the processor 250 may generate the second screen on which an adjustment object for adjusting a treatment parameter of the medical device is displayed, and may control the display 240 so that the display 240 displays (presents) the second screen.

The processor 250 may detect the interaction of a user with an adjustment object of the second screen displayed on the display 240. In this case, the "interaction" may refer to a touch.

The processor 250 may adjust a treatment parameter corresponding to the corresponding adjustment object based on an input value corresponding to the detected interaction.

The processor 250 may control an operation of the medical device based on the adjusted treatment parameter.

In the above description, whether an interaction event has been generated is determined by using the proximity sensor, NFC, the gyro sensor, and the tension of the cable. However, a separate button may be used, if necessary, in addition to the proximity sensor, NFC, the gyro sensor, and the tension of the cable. For example, after a separate button (not illustrated) is displayed on the display 240, when a user (e.g., a doctor) touches the separate button, the processor 250 may determine that an interaction event of the user has been generated and control the second screen to be displayed on the display 240.

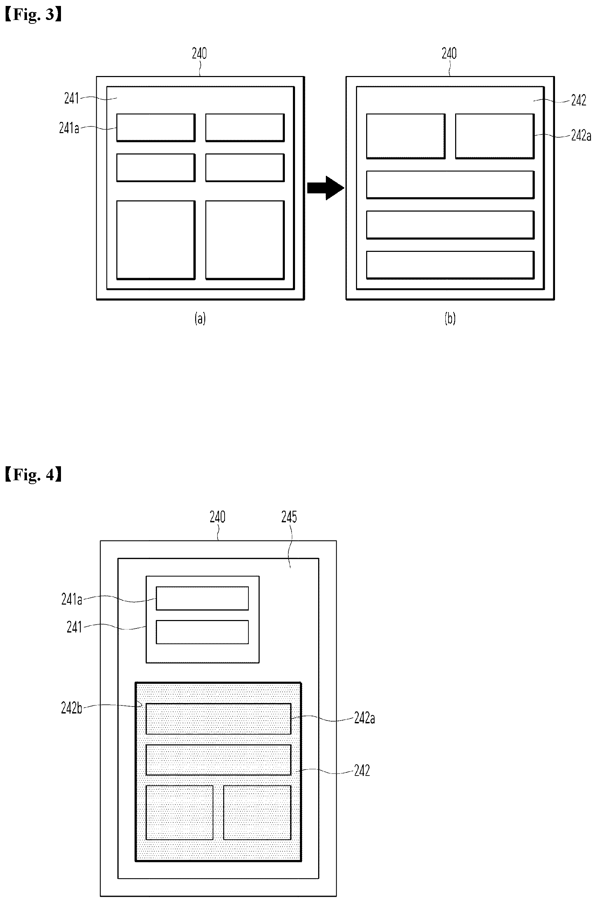

FIGS. 3 to 5 are diagrams exemplarily illustrating the second screen and an adjustment object according to an embodiment of the present disclosure.

Conventionally, both an object of the first screen and an object of the second screen will be displayed on the display 240. When both the object of the first screen and the object of the second screen are displayed on the display 240 as described above, the sizes of the displayed objects are inevitably small due to the limited area of a display area (e.g., a GUI) of the display 240. Accordingly, conventionally, it is necessary to look at the object for a long time or the object is incorrectly pressed because the size of the object is too small. According to an embodiment of the present disclosure, such a problem can be solved.

Referring to FIG. 3, the processor 250 of the controller 200 may generate an object to be displayed on the display 240. In this case, the object includes an adjustment object and a state object.

When the medical device is first turned on, the processor 250 generates a first screen on which one or more state objects 241a are displayed and displays the first screen on the display 240. Accordingly, for example, a first screen 241, such as that illustrated in FIG. 3(a), may be displayed on the display 240.

Thereafter, when determining that an interaction event of a user has been generated, the processor 250 may generate a second screen 242 on which one or more adjustment objects 242a are displayed, and may display the second screen 242 on the display 240.

The processor 250 makes the first screen 241 disappear from the display 240, adjusts the size of an adjustment object of the second screen to be greater than the size of a state object of the first screen, and displays the second screen 242 on the display 240. Accordingly, for example, the second screen 242 illustrated in FIG. 3(b) may be displayed in the entire display area of the display 240. "Making the first screen 241 disappear" is for describing that the first screen 241 switches to the second screen 242. It is said that the first screen 241 is made to disappear from the display 240 because it is preferred that the first screen 241 is not visible when the second screen 242 is displayed on the display 240.

When it is determined that the interaction event of the user has been generated, the second screen 242 has only to be displayed on the display 240. Accordingly, the second screen 242 may be displayed on the display 240 after the first screen 241 is made to disappear, or the first screen 241 may be overlaid with the second screen 242 so that the first screen 241 is made to be invisible.

In FIG. 3, the state objects 241a displayed on the first screen 241 merely show values, but a value according to a manipulation cannot be set.

For example, the state objects 241a displayed on the first screen 241 do not include a "shot count" indicative of the current number of shots upon skin treatment, "delivered" indicative of a total amount of energy, "auto fit" indicative of an output value that is automatically adjusted for each portion of the skin, and "ready/standby" indicating that a current state is a treatment-possible state or a treatment-impossible state.

In FIG. 3, the adjustment objects 242a displayed in the second screen 242 are capable of an interaction. A treatment parameter corresponding to a corresponding adjustment object may be adjusted (or changed) by a manipulation (e.g., a touch) of a user.

For example, the adjustment objects 242a displayed in the second screen 242 may include "count reset" by which a shot count can be reset, a mode (Modes 1 to 10) in which a desired mode (e.g., a treatment mode) can be selected and changed, a level (Levels 1 to 10) at which a desired level (e.g., a vibration level) can be selected and changed, and cooling (Cooling 1 to 10) in which desired cooling (e.g., a cooling level) can be selected and changed.

Although not illustrated in FIG. 3, predetermined letters and/or numbers may be displayed in the area of the state objects 241a of the first screen 241 and the area of the adjustment object 242a of the second screen 242.

Referring to FIG. 3, when an interaction event of a user (e.g., a doctor) who approaches the controller 200 is generated during a skin treatment, the second screen 242 on which the adjustment object (i.e., an active object) 242a for adjusting a treatment parameter of the medical device is displayed on the display 240 under the control of the processor 250. In this case, the size of the adjustment object 242a is greater than the size of the state object 241a of the first screen 241. That is, the adjustment object 242a of the second screen 242 is better visible and a case in which the adjustment object 242a of the second screen 242 is erroneously touched is reduced because the size of the adjustment object 242a of the second screen 242 is great compared to a conventional case. Accordingly, a user can interact with (e.g., touch) a desired adjustment object 242a more easily and conveniently.

Referring to FIG. 4, the processor 250 of the controller 200 may generate an object to be displayed on the display 240. In this case, the object includes an adjustment object and a state object.

When the medical device is first turned on, the processor 250 generate a first screen on which one or more state objects 241a are displayed, and displays the first screen on the display 240. Accordingly, for example, a first screen 241, such as that illustrated in FIG. 3(a), may be displayed on the display 240.

Thereafter, when determining that an interaction event of a user has been generated, the processor 250 may generate a second screen on which one or more adjustment objects 242a are displayed, and may display the second screen on the display 240. For example, as illustrated in FIG. 4, a second screen 242 may be displayed in a display area 245 of the display 240.

In FIG. 3, the first screen 241 is made to disappear (i.e., invisible). In contrast, in FIG. 4, it has been exemplified that the first screen 241 and the second screen 242 are displayed in one display area 245.

In FIG. 4, the processor 250 disposes the first screen 241 to lean toward some area (e.g., a top left side) in the display area 245 of the display 140 by reducing the size of the first screen 241, adjusts the size of the adjustment object 242a of the second screen 242 to be greater than the size of the state object 241a of the first screen 241, and also adjusts the size of the second screen 242 so that the adjustment objects 242a having the size adjusted properly appears in the second screen 242. That is, the size of the adjustment object 242a of the second screen 242 is greater than the size of the state object 241a of the first screen 241, and the size of the second screen 242 is also greater than the size of the first screen 241. Accordingly, the first screen 241 and the second screen 242 are displayed in the display area 245 of the display 240.

Although not illustrated in FIG. 4, predetermined letters and/or numbers may be displayed in the areas of the state object 241a of the first screen 241 and the adjustment object 242a of the second screen 242.

The processor 250 may search the display area 245 of the display 240 for an edge 242b of the second screen 242 in response to an interaction event from a user, and may indicate the edge 242b in a set color (e.g., red or thick black). This is for visually highlighting the second screen 242 and/or the adjustment object 242a. The second screen 242 and/or the adjustment object 242a can be identified more easily. That is, when the interaction event of the user is generated, the user interacts (e.g., touches) a desired adjustment object. The indication of the edge is to enable a user to easily identify which second screen 242 is to interact with and where the adjustment object is located.

Furthermore, the processor 250 may search the display area 245 of the display 240 for the edge 242b of the second screen 242 in response to an interaction event from a user, and may display the remaining area of the second screen 242 except the edge 242b in a set color (e.g., yellow). This is also for visually highlighting the second screen 242 and/or the adjustment objects 242a. Accordingly, the second screen 242 and/or the adjustment objects 242a can be identified more easily. In this case, although the remaining area of the second screen 242 except the edge 242b is indicated in the set color, it is preferred that letters and/or numbers of each adjustment object 242a can be checked by the naked eye. Accordingly, what the adjustment object 242a means can be easily identified by the user.

In order to visually highlight the second screen 242 and/or an adjustment object of the second screen 242, the processor 250 may adopt one or more of a construction in which the edge 242b of the second screen 242 is indicated in a set color and construction in which the remaining area of the second screen 242 except the edge 242b is indicated in a set color.

Referring to FIG. 4, when an interaction event of a user (e.g., a doctor) who approaches the controller 200 during a skin treatment, both the first screen 241 on which an object (i.e., a passive object) related to an operation of the medical device is displayed and the second screen 242 on which an adjustment object (i.e., an active object) for adjusting a treatment parameter of the medical device is displayed are displayed on the display 240 under the control of the processor 250. Although both the first screen 241 and the second screen 242 are displayed, the adjustment object 242a is better visible and can be manipulated more easily because the size of the adjustment object 242a of the second screen 242 is greater than the size of the state object 241a of the first screen 241 and the size of the second screen 242 is also greater than the size of the first screen 241. Furthermore, the areas of the second screen 242 and the adjustment object 242a can be well identified and manipulated more easily because the edge 242b of the second screen 242 is indicated in a set color and the remaining area of the second screen 242 except the edge 242b is indicated in a set color. Accordingly, a user can reduce a case in which the user erroneously manipulates a desired adjustment object 242a and can interact (e.g., touch) a desired adjustment object easily and conveniently.

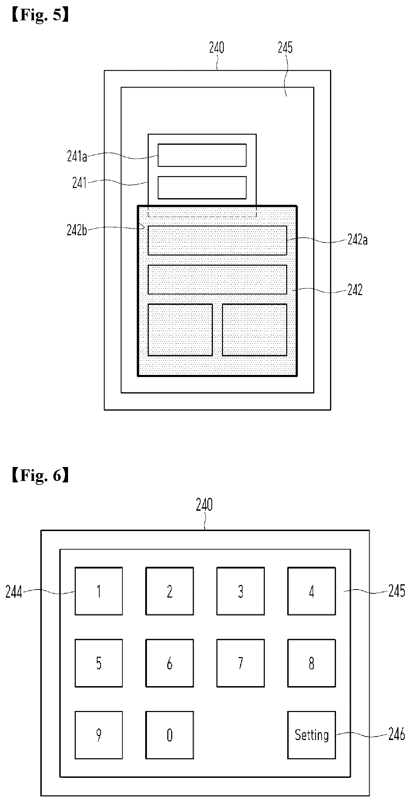

FIG. 5 is almost the same as FIG. 4 except that at least some area of the first screen 241 is overlaid with the second screen 242. In the following description given with reference to FIG. 5, only a difference is described and the remaining description is replaced with the description of FIG. 4. In FIG. 5, the processor 250 controls the display 240 so that at least some area of the first screen 241 is overlaid with the second screen 242 in the display area 245 of the display 240. In FIG. 5, some area of a lower part of the first screen 241 has been overlaid with the second screen 242, but the area of the overlaid area may be larger than the overlay area in FIG. 5. Furthermore, as some area of the lower part of the first screen 241 is overlaid with the second screen 242, the overlaid area of the first screen 241 may not be visible in the display 240.

Referring to FIG. 5, when an interaction event of a user (e.g., a doctor) who approaches the controller 200 is generated during a skin treatment, both the first screen 241 and the second screen 242 are displayed on the display 240 under the control of the processor 250. In this case, the adjustment object 242a is better visible and can be manipulated more easily because the size of the adjustment object 242a of the second screen 242 is greater than the size of the state object 241a of the first screen 241, the size of the second screen 242 is also greater than the size of the first screen 241, and some area of the lower part of the first screen 241 is overlaid with the second screen 242. Furthermore, the areas of the second screen 242 and the adjustment object 242a can be better identified and the adjustment object 242a can be manipulated more easily because the edge 242b of the second screen 242 is indicated in a set color and the remaining area of the second screen 242 except the edge 242b is indicated in a set color. Accordingly, a user can reduce a case in which the user erroneously manipulates a desired adjustment object 242a and can interact (e.g., touch) the adjustment object 242a more conveniently.

The second screen 242 may be generated slightly differently from the contents described with reference to FIGS. 3 to 5.

For example, when the second screen 242 is generated, at least some of the state objects 241a of the first screen 241 may be visually changed, and the second screen may include the visually changed some objects of the first screen. That is, the adjustment object of the second screen 242 may be constructed by changing at least some of the state objects 241a of the first screen 241 into the adjustment objects 242a.

In other words, when the second screen 242 is generated, the second screen 242 may be constructed by including new objects quite different from the state objects 241a of the first screen 241 in the second screen 242 as the adjustment objects 242a and also changing at least some of the state objects 241a of the first screen 241 into the adjustment objects 242a.

FIG. 6 is a diagram for describing a method of manipulating an adjustment object according to an embodiment of the present disclosure.

In an embodiment of the present disclosure, a plurality of input value selection buttons in which different input values have been set has been assigned to the adjustment object, and the plurality of input values set in the plurality of input value selection buttons assigned to the adjustment object, respectively, have been stored in the memory 230. In this case, the plurality of input value selection buttons may each be an example of the treatment adjustment object.

Accordingly, when a user selects (or touches) any one of adjustment objects of the second screen, the processor 250 provides the screen data of a plurality of input value selection buttons to the display 240 so that the plurality of input value selection buttons assigned to the selected adjustment object is displayed on the display 240.

Accordingly, as illustrated in FIG. 6, the display 240 may display a plurality of input value selection buttons 244 in the display area 245 of the display 240. In this case, the sizes of the plurality of input value selection buttons 244 are adjusted and displayed in order to provide the easiness of identification and selection. For example, in FIG. 6, the number of input value selection buttons 244 is assumed to be ten. In order for each of the input value selection buttons 244 to be easily identified and directly selected, the processor 250 enlarges the size of each of the input value selection buttons 244 and then displays the plurality of input value selection buttons 244 in the display area 245 of the display 240 so that the plurality of input value selection buttons 244 is spaced apart from each other. That is, it may be said that the screen data of the plurality of input value selection buttons include information on the size of each of the input value selection buttons 244 each having the size enlarged. The screen data of the plurality of input value selection buttons may further include location information in addition to information on the size of each input value selection button 244, if necessary. The ten input value selection buttons 244 having the sizes enlarged are distributed and displayed to the extent that the ten input value selection buttons cover the entire display area 245. FIG. 6 exemplifies that a setting button 246 is additionally included, but the setting button 246 may not be included, if necessary.

Referring to FIG. 6, when a user (e.g., a doctor) directly selects (or touches) a desired button, among the plurality of input value selection buttons 244, the processor 250 adjusts a treatment parameter corresponding to a corresponding adjustment object based on an input value that is set in the corresponding button (i.e., the selected button) in response to the selection. That is, the processor 250 may read an input value set in an input value selection button that is currently selected from the memory 230 because the input value set in each of the plurality of input value selection buttons 244 is stored in the memory 230, and may adjust a treatment parameter corresponding to a corresponding adjustment object based on the read input value. For example, assuming that a user has selected (or touched) a button indicated as "5", among the plurality of input value selection buttons 244, the processor 250 determines an input value to be "5". Accordingly, the processor 250 adjusts a treatment parameter corresponding to a corresponding adjustment object as "5".

Thereafter, the processor 250 controls an operation of the medical device based on the adjusted treatment parameter. For example, when an adjustment object is shot intensity, the processor 250 adjusts shot intensity as "5" and enables a skin treatment to be performed based on the adjusted shot intensity.

In the method of FIG. 6, buttons by which input values can be selected can be intuitively identified because the plurality of input value selection buttons 244 assigned to a selected adjustment object is displayed on the display 240 when any one of the adjustment objects 242a of the second screen 242 is selected (or touched). Accordingly, there is an effect in that a desired input value can be input very easily and rapidly because the desired input value can be directly selected and input.

Furthermore, there is an effect in that a desired input value can be input accurately and rapidly compared to a sliding or arrow method for selecting and inputting an input value.

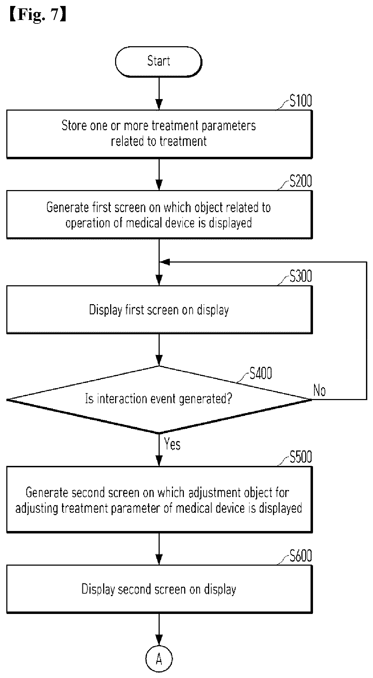

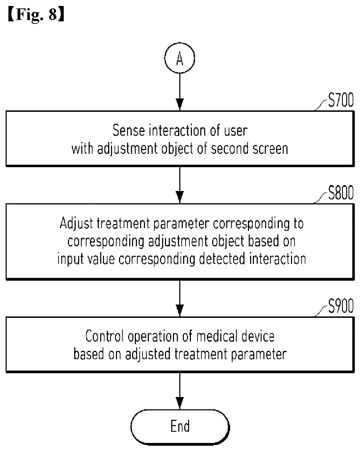

FIGS. 7 and 8 are flowcharts for describing a screen display method for a medical device according to an embodiment of the present disclosure.

The processor 250 stores one or more treatment parameters related to a treatment in the memory 230 (S100).

When an operation of the medical device (e.g., a skin treatment device) is turned on, the processor 250 generates a first screen on which an object related to the operation of the medical device is displayed (S200).

Thereafter, the processor 250 controls the display 240 so that the display 240 displays the first screen. Accordingly, for example, a first screen, such as that in FIG. 3(a), may be displayed on the display 240 of the controller 200 (S300).

Accordingly, the processor 250 controls a skin treatment for a patient to be performed based on information of the object related to the operation of the medical device, which is displayed on the display 240.

Thereafter, the processor 250 determines whether an interaction event of a user (e.g., a doctor) has been generated (S400).

For example, when a user (e.g., a doctor) approaches the controller 200 in order to change a treatment parameter (e.g., a value of shot intensity) during a skin treatment, the sensing module 210 senses the approach of at least body part of the user. The processor 250 may determine that an interaction event has been generated based on an approach sensing signal from the sensing module 210.

When determining that the interaction event of the user has been generated as described above, the processor 250 generates a second screen on which an adjustment object for adjusting the treatment parameter of the medical device is displayed (S500).

Thereafter, the processor 250 controls the display 240 so that the display 240 displays the second screen. Accordingly, for example, a second screen, such as that in FIG. 3(b), may be displayed on the display 240 of the controller 200. Unlike in FIG. 3(b), as illustrated in FIG. 4 or 5, the first screen 241 and the second screen 242 may be displayed in the display area 245 of the display 240 (S600).

The processor 250 senses the interaction of the user with the adjustment object of the second screen displayed on the display 240 (S700). For example, when a user selects (or touches) any one of the adjustment objects of the second screen, a corresponding touch signal is generated by the input module 220 and applied to the processor 250. Accordingly, the processor 250 provides the display 240 with the screen data of a plurality of input value selection buttons so that a plurality of input value selection buttons assigned to the adjustment object that is currently selected is displayed on the display 240 based on the received touch signal. Accordingly, as illustrated in FIG. 6(b), the display 240 displays the plurality of input value selection buttons 244 in the display area 245 of the display 240. In this state, when a user (e.g., a doctor) directly selects (or touches) a desired button, among the plurality of input value selection buttons 244, the input module 220 generates a signal (e.g., may be said to be a button identification signal) that provides notification that the selected button corresponds to any button and applies the signal to the processor 250. The processor 250 senses which button has been selected based on the received button identification signal.

Thereafter, the processor 250 reads an input value set in the input value selection button that is currently selected from the memory 230, and adjusts the treatment parameter corresponding to the corresponding adjustment object based on the read input value (S800).

Thereafter, the processor 250 controls an operation of the medical device based on the adjusted treatment parameter (S900).

The above description is merely a description of the technical spirit of the present disclosure, and a person having ordinary knowledge in the field to which the present disclosure pertains may change and modify the present disclosure in various ways without departing from the essential characteristic of the present disclosure. Accordingly, the embodiments disclosed in the present disclosure should not be construed as limiting the technical spirit of the present disclosure, but should be construed as describing the technical spirit of the present disclosure. The range of the technical spirit of the present disclosure is not restricted by the embodiments. The range of protection of the present disclosure should be construed based on the following claims, and all of technical spirits within an equivalent range of the present disclosure should be construed as being included in the scope of rights of the present disclosure.

### [Description of reference numerals]

| | | | |
|---|---|---|---|
| 100: | handpiece | 200: | controller |
| 210: | sensing module | 220: | input module |
| 230: | memory | 240: | display |
| 241: | first screen | 242: | second screen |
| 245: | display area | 250: | processor |
| 300: | cable | 210a: | proximity sensor |

## Claims

1. A screen display apparatus for a medical device comprising a handpiece for a treatment, the screen display apparatus comprising:
a display;
memory in which one or more treatment parameters related to the treatment are stored; and
a processor configured to control an operation of the medical device based on the treatment parameter,
wherein the processor
generates a first screen which presents a state object related to the operation of the medical device,
controls the display so that the display presents the first screen,
determines whether an interaction event for the medical device is generated,
generates a second screen which presents an adjustment object for adjusting the treatment parameter of the medical device when determining that the interaction event has been generated,
controls the display so that the display presents the second screen,
senses an interaction of a user with the adjustment object of the second screen,
adjusts the treatment parameter corresponding to the adjustment object based on an input value corresponding to the sensed interaction, and
controls the operation of the medical device based on the adjusted treatment parameter.

2. The screen display apparatus of claim 1, wherein the processor senses a proximity of the user and determines that the interaction event has been generated when sensing that the user has approached.

3. The screen display apparatus of claim 1, wherein when determining that the interaction event has been generated, the processor controls the first screen to disappear from the display and controls the second screen to be presented on the display.

4. The screen display apparatus of claim 3, wherein the processor adjusts a size of the adjustment object of the second screen to be greater than a size of the state object of the first screen on the display.

5. The screen display apparatus of claim 1, wherein when determining that the interaction event has been generated, the processor controls the first screen and the second screen to be presented on the display so that the first screen and the second screen are spaced apart from each other.

6. The screen display apparatus of claim 5, wherein the processor adjusts a size of the adjustment object of the second screen to be greater than a size of the state object of the first screen on the display and adjusts a size of the second screen to be greater than a size of the first screen.

7. The screen display apparatus of claim 1, wherein when determining that the interaction event has been generated, the processor controls the first screen and the second screen to be presented on the display and controls at least some area of the first screen to be overlaid with the second screen.

8. The screen display apparatus of claim 7, wherein the processor adjusts a size of the adjustment object of the second screen to be greater than a size of the state object of the first screen and adjusts a size of the second screen to be greater than a size of the first screen on the display.

9. The screen display apparatus of claim 1, wherein the processor displays an edge of the second screen in a set color in response to the interaction event.

10. The screen display apparatus of claim 1, wherein the processor controls the display to present a remaining area of the second screen except an edge of the second screen in a set color in response to the interaction event.

11. The screen display apparatus of claim 1, wherein:
a plurality of input value selection buttons in which different input values have been set is assigned to the adjustment object, and
the processor provides the display with screen data of the plurality of input value selection buttons so that the plurality of input value selection buttons assigned to the adjustment object is presented on the display when the adjustment object is selected.

12. The screen display apparatus of claim 11, wherein when any one of the plurality of input value selection buttons is selected, the processor adjusts the treatment parameter corresponding to the adjustment object based on an input value set in the selected button.

13. A screen display method performed by a screen display apparatus for a medical device comprising a handpiece for a treatment, the screen display method comprising:
storing one or more treatment parameters related to the treatment;
generating a first screen which presents a state object related to an operation of the medical device;
controlling a display so that the display presents the first screen;
determining whether an interaction event for the medical device is generated;
generating a second screen which presents an adjustment object for adjusting a treatment parameter of the medical device when determining that the interaction event has been generated;
controlling the display so that the display presents the second screen;
sensing an interaction of a user with the adjustment object of the second screen;
adjusting the treatment parameter corresponding to the adjustment object based on an input value corresponding to the detected interaction; and
controlling the operation of the medical device based on the adjusted treatment parameter.
